# EUROPEAN PATENT APPLICATION

(11) **EP 1 967 322 A1**
(43) Date of publication of application: **10.09.2008**
(21) Application number: 05822739.8
(22) Date of filing: 27.12.2005
(51) Int. Cl.: B25B 1/24, B25B 1/02, F16B 2/12, B25B 5/02

(54) **UNIVERSAL GRIPPING TOOL FOR OPERATION TABLE**

(71) Applicant: Iso Medical Systems Inc., Tokyo 113-0033 (JP)
(72) Inventor: ISO, Tsutomu, Abiko-shi, Chiba 270-1132 (JP)
(74) Representative: Manley, Nicholas Michael
(86) International application number: PCT/JP2005/024234
(87) International publication number: WO 2007/074532

(57) **Abstract**

An object of the present invention is to provide a universal fixing metal fitting 1 for an operating table capable of being easily attached and detached, capable of enabling simultaneous firm fixing between the fixing metal fitting, an appliance to be fixed, and an accessory attachment table by performing fastening one time regardless of the existence/nonexistence of a sheet or the like, capable of ensuring the same firm fixing even when the shape of the accessory attachment table is changed and capable of fixing no matter what the shape of the appliance to be fixed is. To achieve the above-described object, there is provided a universal fixing metal fitting 1 for an operating table **characterized by** having a base member 2 in which a first attachment leg 2b having an attachment portion 2d and including a slant portion 2h formed thereon and a second attachment leg 2c having an attachment portion 2e and including a slant portion 2i formed thereon are formed continuously with a flat portion 2a having a screw-engaging hole 2k formed at its center, an upper pinching member 3 in which a first pinching portion 3b is formed continuously with one end of a connecting portion 3a having a V-cut 3k at its center and L-cuts 3d formed continuously with the V-cut 3k, and in which a second pinching portion 3c is formed continuously with the other end of the connecting portion 3a, a lower pinching member 4 in which a third pinching portion 4b is formed continuously with one end of a connecting portion 4a having a V-cut 4k at its center and L-cuts 4d formed continuously with the V-cut 4k, and in which a fourth pinching portion 4c is formed continuously with the other end of the connecting portion 4a, and a fastening portion 5 in which a handle 5c having spherical stoppers 5d provided on its opposite ends is loosely fitted in a through hole 5f in a cylindrical head portion 5a provided on one end of a screw-engaging shaft 5b, the universal fixing metal fitting 1 also **characterized in that** when the upper pinching member 3 and the lower pinching member 4 are coupled to the base member 2, coil springs are attached by being interposed between the first pinching portion 3b and the attachment portion 2d, between the second pinching portion 3c and the attachment portion 2e, between the third pinching portion 4b and the attachment portion 2d and between the fourth pinching portion 4c and the attachment portion 2e, and in that the screw-engaging shaft 5b is mounted in the screw-engaging hole 2k when the fastening portion 5 is coupled to the base member 2. There is also provided a universal fixing metal fitting 1 for an operating table having the above-described features and also **characterized in that** a pressing member 5e is attached to a tip of the screw-engaging shaft 5b.

## Description

### Technical Field

The present invention relates to a fixing metal fitting for an operating table used when a member, an instrument or the like is mounted in any position on an accessory attachment rail along the operating table.

### Background Art

A subject and an operating table are covered with a clean sheet or the like during surgical operation. It was, therefore, difficult to mount a conventional fixing metal fitting over a sheet or the like in such a case. A type of fixing metal fitting capable of fixing over a sheet or the like was thereafter developed. However, fixing of the fixing metal fitting on an accessory attachment rail and fixing between the fixing metal fitting and an appliance to be fixed were performed separately from each other. A considerably long time was required for mounting, and the fixing metal fitting, the accessory attachment table and the appliance to be fixed were not firmly fixed and stabilized.

A fixing metal fitting was also developed in which, as disclosed in U.S. Patent 5535973, an accessory attachment rail was pinched between upper and lower arms and a flat plate; a knob was fastened to reduce the distance between left and right arms and the flat plate; and the fixing metal fitting and an appliance to be fixed are were simultaneously fixed on an accessory attachment rail by the pressure of the fastening. However, there was still the same difficulty in mounting, the force for fixing to the accessory attachment rail varied depending on the thickness of a sheet or the like, and it was difficult to obtain a sufficient and stable fixing force.

Thus, conventional fixing metal fittings lacked safety and were, therefore, not suitable as a fixing metal fitting for fixing an appliance on an accessory attachment rail of an operating table and were incapable of enabling medical practitioners to do medical work without anxiety.

Also, it was difficult to firmly fix the conventional fixing metal fittings in the case of use with accessory attachment rails differing in shape and an increased number of sheets or the like. Also for this reason, the conventional fixing metal fittings are unsuitable as a medical appliance which needs to be safe.

It is, therefore, an object of the present invention to provide a universal fixing metal fitting for an operating table capable of being easily attached and detached, capable of enabling simultaneous firm fixing between the fixing metal fitting, an appliance to be fixed, and an accessory attachment table by performing fastening one time regardless of the existence/nonexistence of a sheet or the like, capable of ensuring the same firm fixing even when the shape of the accessory attachment table is changed and capable of fixing no matter what the shape of the appliance to be fixed is.

### Disclosure of the Invention

To achieve the above-described object, according to the present invention, there is provided a universal fixing metal fitting 1 for an operating table characterized by having a base member 2 in which a first attachment leg 2b having an attachment portion 2d and including a slant portion 2h formed thereon and a second attachment leg 2c having an attachment portion 2e and including a slant portion 2i formed thereon are formed continuously with a flat portion 2a having a screw-engaging hole 2k formed at its center, an upper pinching member 3 in which a first pinching portion 3b is formed continuously with one end of a connecting portion 3a having a V-cut 3k at its center and L-cuts 3d formed continuously with the V-cut 3k, and in which a second pinching portion 3c is formed continuously with the other end of the connecting portion 3a, a lower pinching member 4 in which a third pinching portion 4b is formed continuously with one end of a connecting portion 4a having a V-cut 4k at its center and L-cuts 4d formed continuously with the V-cut 4k, and in which a fourth pinching portion 4c is formed continuously with the other end of the connecting portion 4a, and a fastening portion 5 in which a handle 5c having spherical stoppers 5d provided on its opposite ends is loosely fitted in a through hole 5f in a cylindrical head portion 5a provided on one end of a screw-engaging shaft 5b having a pressing member 5e attached to the other end, the universal fixing metal fitting 1 also characterized in that when the upper pinching member 3 and the lower pinching member 4 are coupled to the base member 2, coil springs are attached by being interposed between the first pinching portion 3b and the attachment portion 2d, between the second pinching portion 3c and the attachment portion 2e, between the third pinching portion 4b and the attachment portion 2d and between the fourth pinching portion 4c and the attachment portion 2e, and in that the screw-engaging shaft 5b is mounted in the screw-engaging hole 2k when the fastening portion 5 is coupled to the base member 2.

### Brief Description of the Drawings

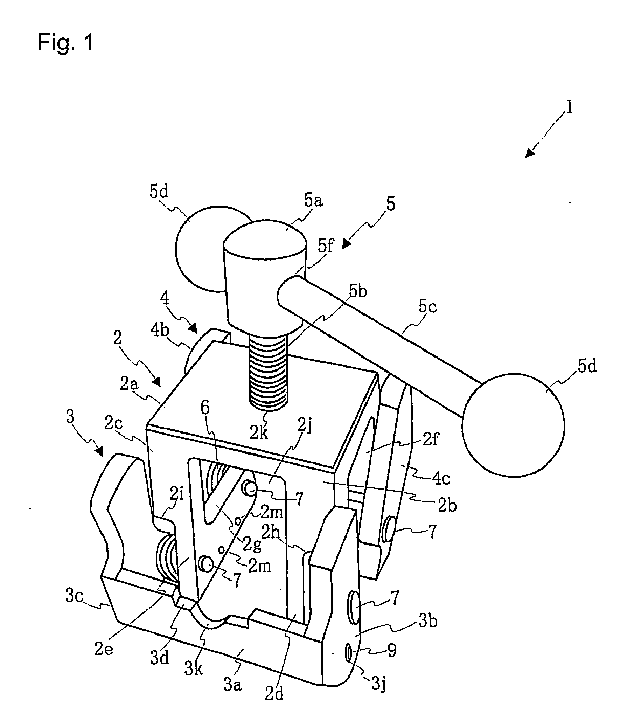

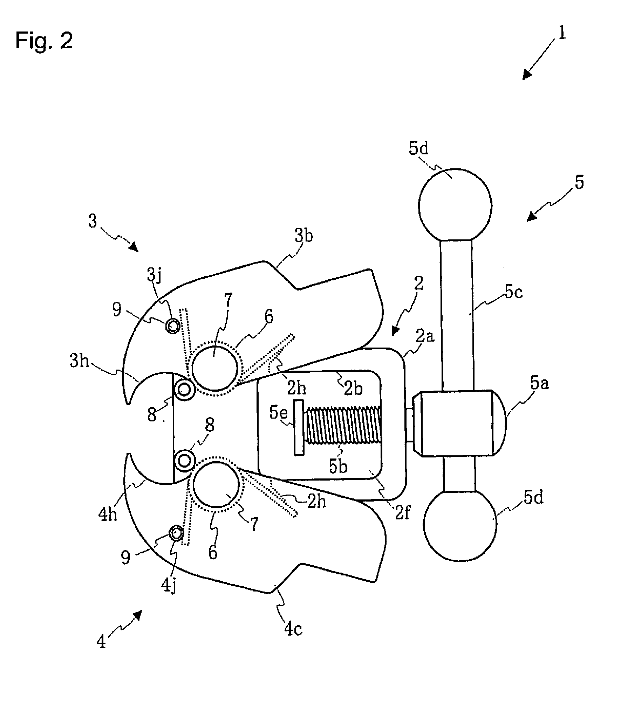

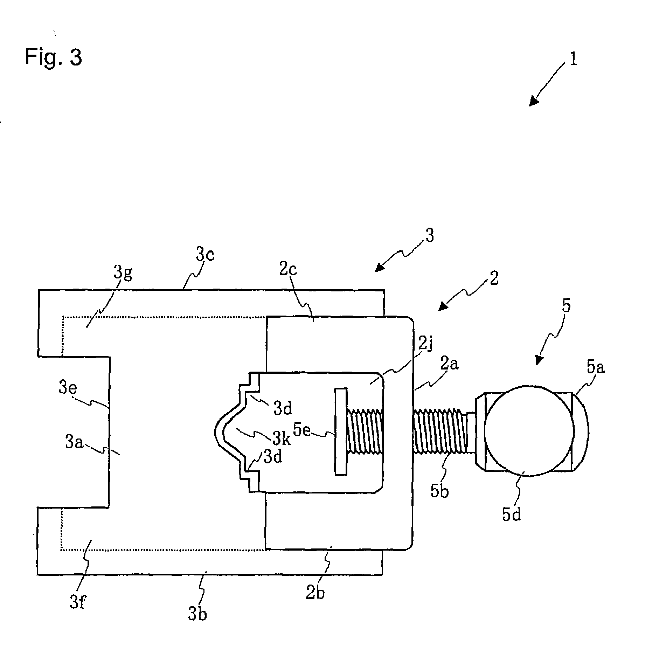

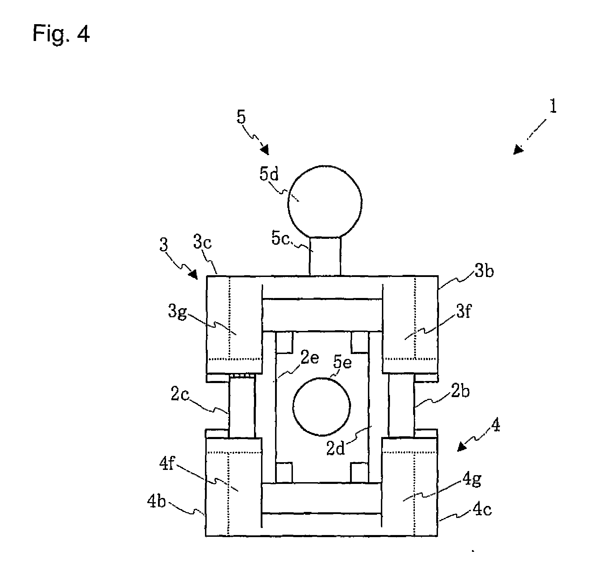

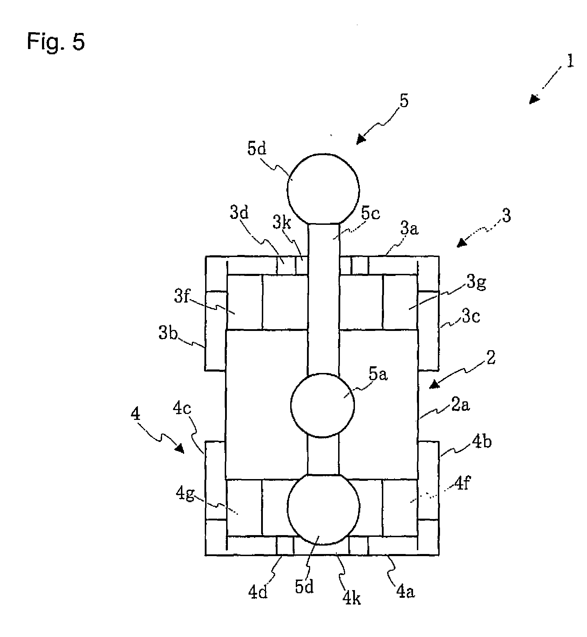

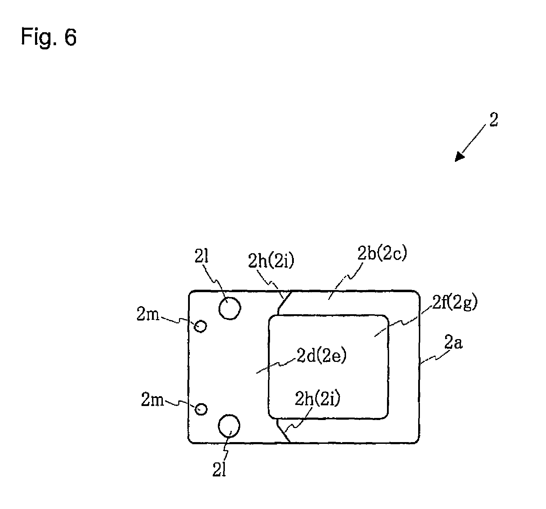

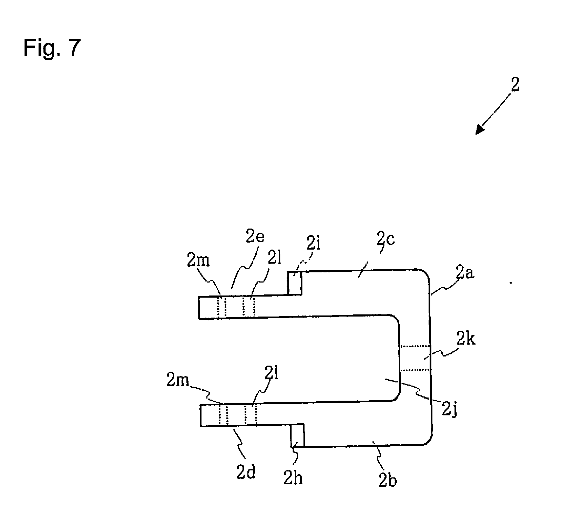

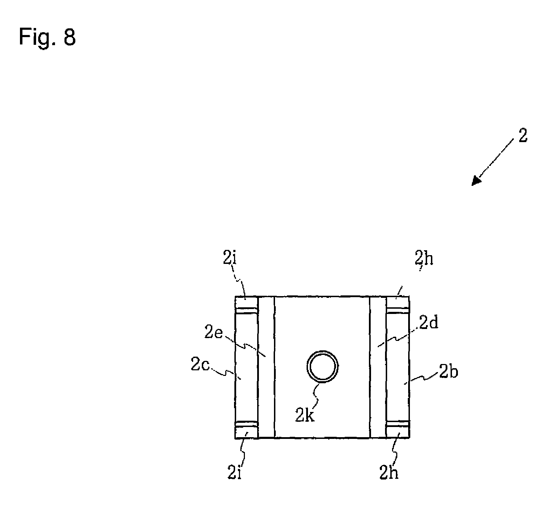

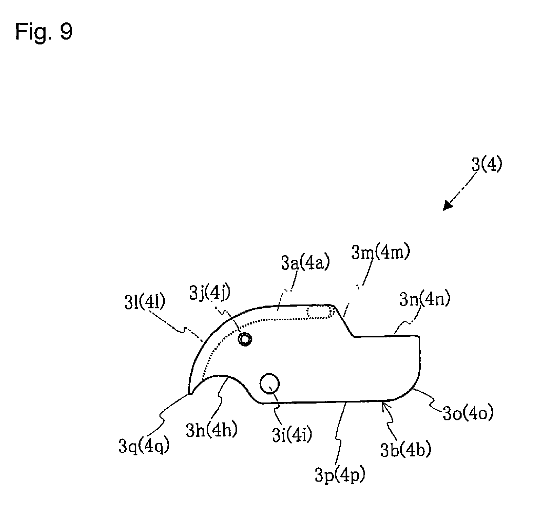

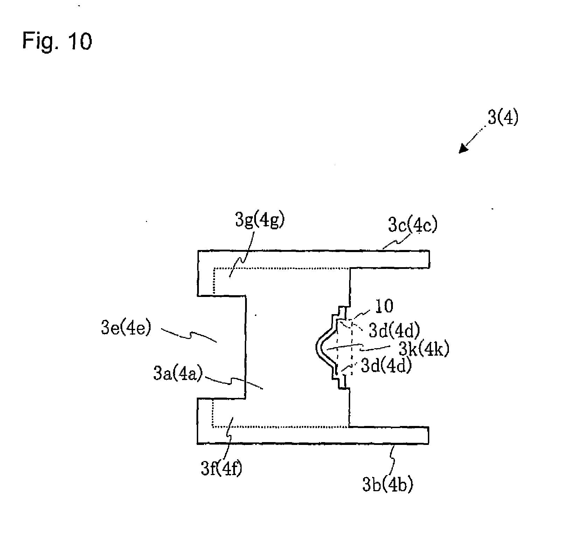

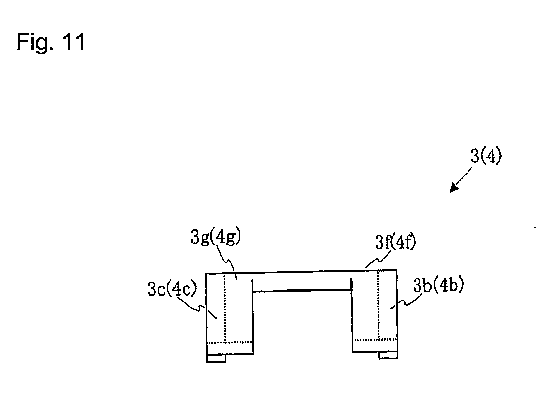

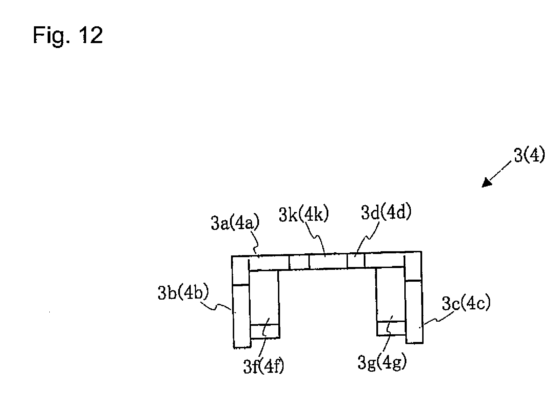

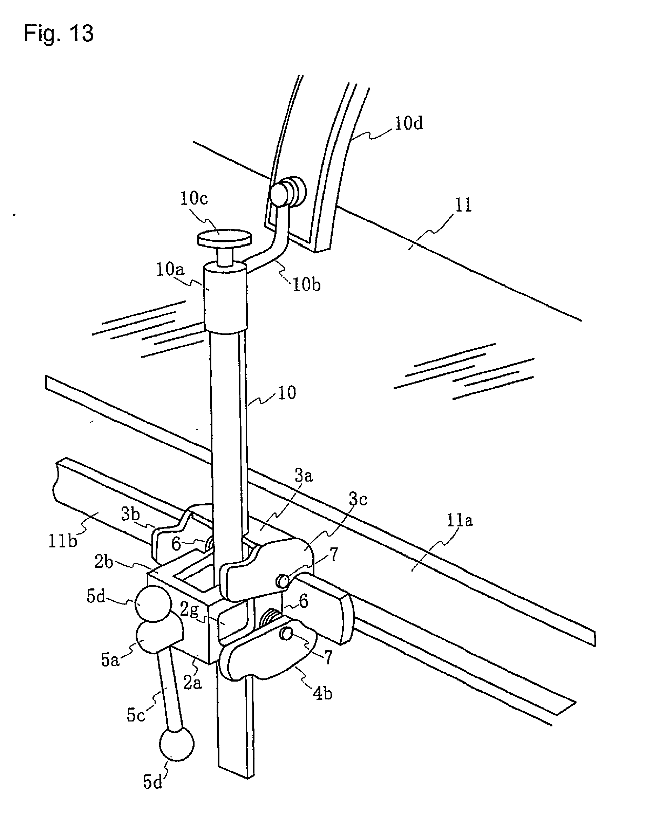

Figure 1 is a perspective view of a universal fixing metal fitting for an operating table according to the present invention; Figure 2 is a front view of the universal fixing metal fitting for an operating table according to the present invention; Figure 3 is a plan view of the universal fixing metal fitting for an operating table according to the present invention; Figure 4 is a left side view of the universal fixing metal fitting for an operating table according to the present invention; Figure 5 is a right side view of the universal fixing metal fitting for an operating table according to the present invention; Figure 6 is a front view of a base member of the universal fixing metal fitting for an operating table according to the present invention; Figure 7 is a plan view of the base member of the universal fixing metal fitting for an operating table according to the present invention; Figure 8 is a left side view of the base member of the universal fixing metal fitting for an operating table according to the present invention; Figure 9 is a front view of upper and lower pinching members of the universal fixing metal fitting for an operating table according to the present invention; Figure 10 is a plan view of the upper pinching member and the lower pinching member of the universal fixing metal fitting for an operating table according to the present invention; Figure 11 is a left side view of the upper pinching member and the lower pinching member of the universal fixing metal fitting for an operating table according to the present invention; Figure 12 is a right side view of the upper pinching member and the lower pinching member of the universal fixing metal fitting for an operating table according to the present invention; and Figure 13 is a diagram showing the state of use of the universal fixing metal fitting for an operating table according to the present invention.

### Best Mode for Carrying Out the Invention

A fixing metal fitting for an operating table according to the present invention will be described in detail with reference to the accompanying drawings.

Figures 1, 2, 3, and 4 are a perspective view, a front view, a plan view, a left side view, and a right side view, respectively, of a universal fixing metal fitting 1 for an operating table according to the present invention. As shown in Figures 1 to 5, the universal fixing metal fitting 1 for an operating table according to the present invention includes a base member 2, an upper pinching member 3, a lower pinching member 4, and a fastening portion 5. The base member 2 includes a flat portion 2a, a first attachment leg 2b formed perpendicularly to and continuously with the flat portion 2a, and a second attachment leg 2c formed perpendicularly to and continuously with the flat portion 2a. The upper pinching member 3 includes a connecting portion 3a, a first pinching portion 3b formed on one end of the connecting portion 3a, and a second pinching portion 3c formed on the other end of the connecting portion 3a.

The lower pinching member 4 includes a connecting portion 4a, a third pinching portion 4b formed on one end of the connecting portion 4a, and a fourth pinching portion 4c formed on the other end of the connecting portion 4a. The fastening portion 5 has as its main components a screw-engaging shaft 5b having a pressing member 5e attached to its tip and a head portion 5a provided on one side thereof, and includes a handle 5c passed through a through hole bored in the head portion 5a and spherical stoppers 5d provided on opposite ends thereof.

As shown in Figure 1, a screw-engaging hole 2k is bored in flat portion 2a of the base member 2 generally in the form of a rectangular block at a center of the same. The screw-engaging shaft 5b is screw-engaged with this screw-engaging hole 2k.

A rectangular through hole 2f is bored in a central portion of the first attachment leg 2b formed perpendicularly to and continuously with the flat portion 2a of the base member 2.

A rectangular through hole 2g is also bored in a central portion of the second attachment leg 2c formed perpendicularly to and continuously with the flat portion 2a.

As shown in Figure 3, at positions closer to the flat portion 2a of the base member 2 in the connecting portion 3a connecting the first pinching portion 3b and the second pinching portion 3c of the upper pinching member 3, L-cuts 3d are respectively formed on the first pinching portion 3b side and the second pinching portion 3c side, and a V-cut 3k is formed between the L-cuts 3d on the opposite sides. The first pinching portion 3b is formed perpendicularly to and continuously with one end of the connecting portion 3a, while the second pinching portion 3c is formed perpendicularly to and continuously with the other end of the connecting portion 3a. The first pinching portion 3b and the second pinching portion 3c are equal in shape and size to each other.

As shown in Figures 4 and 5, the lower pinching member 4 includes a connecting portion 4a in which L-cuts 4d are formed, a third pinching portion 4b formed perpendicularly to and continuously with one end of the connecting portion 4a, and a fourth pinching portion 4c formed perpendicularly to and continuously with the other end of the connecting portion 4a. At positions closer to the flat portion 2a of the base member 2 in the connecting portion 4a connecting the third pinching portion 4b and the fourth pinching portion 4c of the lower pinching member 4, L-cuts 4d are respectively formed on the third pinching portion 4b side and the fourth pinching portion 4c side, and a V-cut 4k is formed between the L-cuts 4d on the opposite sides. Reference characters 3f, 3g, 4f, and 4g denote inwardly projecting projections of the first pinching portion 3b, the second pinching portion 3c, the third pinching portion 4b and the fourth pinching portion 4c.

The third pinching portion 4b is formed perpendicularly to and continuously with one end of the connecting portion 4a, while the fourth pinching portion 4c is formed perpendicularly to and continuously with the other end of the connecting portion 4a. The third pinching portion 4b and the fourth pinching portion 4c are equal in shape and size to each other. Also, the upper pinching member 3 and the lower pinching member 4 are equal in shape and size to each other. Details of the members will be described with reference to Figures 6 to 12 before describing the states of coupling between the members. Figure 6 is a front view of the base member 2 constituting the universal fixing metal fitting for an operating table according to the present invention; Figure 7 a plan view of the base member 2; and Figure 8 a left side view of the base member 2.

As shown in Figures 6 to 8, the base member 2 is a member provided as a basis for the universal fixing metal fitting 1 for an operating table according to the present invention. As shown in Figure 6, the first attachment leg 2b and the second attachment leg 2c are formed perpendicularly to and continuously with the opposite ends of the flat portion 2a of the base member 2, and the through holes 2f and 2g are formed in central portions of these attachment legs.

As shown in Figure 7, slant portions 2h and 2i are provided at intermediate positions in the first attachment leg 2b and the second attachment leg 2c, and an attachment portion 2d and an attachment portion 2e provided on the left hand sides of the slant portions 2h and 2i continuously with the slant portions 2h and 2i are formed so that their thickness is about half that of the portions on the right hand sides. The slant portions 2h and 2i are provided on the outer surface sides of the attachment portion 2d and the attachment portion 2e. Accordingly, the inner wall surfaces of the first attachment leg 2b and the second attachment leg 2c are formed as flat surfaces.

As shown in Figures 6 and 7, attachment holes 21 and pin insertion holes 2m are bored in upper and lower portions in the attachment portion 2d of the first attachment leg 2b and the attachment portion 2e of the second attachment leg 2c. The four pin insertion holes 2m in total are small holes, while the four attachment holes 21 in total are large holes.

As shown in Figures 6 to 8, the slant portions 2h are formed in two places in the first attachment leg 2b, and the slant portions 2i are formed in two places in the second attachment leg 2c. The slant portions 2h and 2i slant toward centers of surfaces of the attachment portion 2d and the attachment portion 2e shown in Figure 6.

As shown in Figures 9 to 12, the upper pinching member 3 and the lower pinching member 4 are equal in shape and size to each other. Therefore, description will be made below mainly of the upper pinching member 3 and description will then be made of the lower pinching member 4.

Referring to Figure 10, the upper pinching member 3 includes the connecting portion 3a, the first pinching portion 3b formed continuously with one end of the connecting portion 3a, and the second pinching portion 3c formed continuously with the other end of the connecting portion 3a.

As shown in Figure 9, a left end of the first pinching portion 3b is formed generally into a bird's beak shape; an upper curved portion 31 which curves largely toward an extreme end 3q is formed in an upper left position; a slant portion 3m is formed continuously from the upper curved portion 31 to the right; and an upper flat portion 3n is formed continuously from the slant portion 3m to the right.

A U-cut 3h is provided on the right hand side of the extreme end 3q of the first pinching portion 3b, and a lower flat portion 3p is formed continuously from the U-cut 3h to the right. A lower curved portion 3o is formed continuously from the lower flat portion 3p to the right. A coupling hole 3i is bored on the right hand side of the U-cut 3h, and a pin insertion hole 3j is bored in the vicinity of the upper curved portion 31.

The upper pinching member 3 is of such a structure that the connecting portion 3a, the first pinching portion 3b and the second pinching portion 3c are not provided as separate members but formed integrally with each other.

As shown in Figure 10, a rectangular recess 3e is formed on the left hand side of the connecting portion 3a connecting the first pinching portion 3b and the second pinching portion 3c on the opposite ends.

The L-cuts 3d are formed on the right hand side of the connecting portion 3a in two places at opposed positions such that an attachment rod 10 having a square or rectangular section can be firmly fixed so as not to swing. The V-cut 3k is formed between the L-cut 3d and the L-cut 3d in the two places to enable fixation of a cylindrical member.

Each of the upper pinching member 3 and the lower pinching member 4 has is generally H-shaped.

As shown in Figures 11 and 12, the left and right side surfaces of the upper pinching member 3 constitute a generally U-shaped configuration.

As shown in Figures 9 to 12, the lower pinching member 4 includes the connecting portion 4a, the third pinching portion 4b formed continuously with the right end of the connecting portion 4a, and the fourth pinching portion 4c formed continuously with the left end of the connecting portion 4a.

As shown in Figure 9, the third pinching portion 4b is generally of a bird's beak shape; an upper curved portion 41 which curves largely toward an extreme end 4q is formed in an upper left position; a slant portion 4m is formed continuously from the upper curved portion 41 to the right; and an upper flat portion 4n is formed continuously from the slant portion 4m to the right.

A U-cut 4h is provided on the right hand side of the extreme end 4q of the third pinching portion 4b, and a lower flat portion 4p is formed continuously from the U-cut 4h to the right. A lower curved portion 4o is formed continuously from the lower flat portion 4p to the right. A coupling hole 4i is bored on the right hand side of the U-cut 4h, and a pin insertion hole 4j is bored in the vicinity of the upper curved portion 41.

The lower pinching member 4 is of such a structure that the connecting portion 4a, the third pinching portion 4b and the fourth pinching portion 4c are not provided as separate members but formed integrally with each other.

As shown in Figure 10, a rectangular recess 4e is formed on the left hand side of the connecting portion 4a formed continuously with the third pinching portion 4b and the fourth pinching portion 4c on the opposite ends.

The L-cuts 4d are formed on the right hand side of the connecting portion 4a in two places at opposed positions such that an attachment rod 10 having a square or rectangular section can be firmly fixed so as not to swing. The V-cut 4k is formed between the L-cut 4d and the L-cut 4d in the two places to enable fixation of a cylindrical member.

Each of the upper pinching member 3 and the lower pinching member 4 has is generally H-shaped.

Description will be made below of how the base member 2, the upper pinching member 3, the lower pinching member 4 and the fastening portion 5 are coupled to form the universal fixing metal fitting 1 for an operating table according to the present invention.

As shown in Figures 1 to 5, the screw-engaging shaft 5b having the pressing member 5e attached to its tip is screw-engaged with the screw-engaging hole 2k bored in the flat portion 2a of the base member 2 at a center of the same.

The screw-engaging shaft 5b has the shape of a male screw. A female screw which meshes with the male screw on the screw-engaging shaft 5b is formed in the inner wall surface of the screw-engaging hole 2k.

The pressing member 5e may not be attached to the tip of the screw-engaging shaft 5b. The material and shape of the pressing member 5e are not limited to particular ones. The pressing member 5e may be formed by changing the material and shape as required.

An insertion hole 5f is formed in the head portion 5a attached to the screw-engaging shaft 5b. The handle 5c having the spherical stoppers 5d attached to its opposite ends is loosely inserted in the insertion hole 5f so as to be slidable in one direction. Since the stoppers 5d are attached to the opposite ends of the handle 5c, the handle 5c does not come off the insertion hole 5f of the head portion 5a.

As shown in Figure 2, the upper pinching member 3 and the lower pinching member 4 are turnably attached to the first attachment leg 2b and the second attachment leg 2c of the base member 2 by four retaining members 7.

That is, the upper pinching member 3 is turnably attached to the base member 2 by using the retaining members 7 in the attachment holes 21 provided in the attachment portion 2d of the first attachment leg 2b and the attachment portion 2e of the second attachment leg 2c (see Figure 6) and the coupling hole 3i of the first pinching portion 3b (see Figure 9).

Similarly, the lower pinching member 4 is turnably attached to the base member 2 by using the retaining members 7 in the attachment holes 2l provided in the attachment portion 2d of the first attachment leg 2b and the attachment portion 2e of the second attachment leg 2c (see Figure 7) and the coupling hole 4i of the third pinching portion 4b (see Figure 9).

When the first pinching portion 3b of the upper pinching member 3 and the attachment portion 2d of the first attachment leg 2b are coupled to each other by the retaining member 7, the retaining member 7 is inserted in a coil spring 6 and the coil spring 6 is placed between the first pinching portion 3b and the attachment portion 2d of the first attachment leg 2b.

One end of the coil spring 6 is engaged with the slant portion 2h formed on the first attachment leg 2b, while the other end of the coil spring 6 is engaged with a spring stopper 9 mounted in the pin insertion hole 3j of the first pinching portion 3b.

The range of turning of the first pinching portion 3b is limited by a pin 8 mounted in the pin insertion hole 2m bored in the attachment portion 2b of the first attachment leg 2b.

As shown in Figure 2, when the upper pinching member 3 is turned clockwise on the retaining member 7, the spring stopper 9 inserted in the pin insertion hole 3j and the slant portion 2h are brought closer to each other and the opposite ends of the coil spring are also brought closer to each other. A counterclockwise torque is then produced on the upper pinching member 3 by the restoring force of the coil spring 6 acting to return to the original state. Also, when the second pinching portion 3c of the upper pinching member 3 and the attachment portion 2e of the second attachment leg 2c are coupled to each other by the retaining member 7, the retaining member 7 is inserted in a coil spring 6 and the coil spring 6 is placed between the second pinching portion 3c and the attachment portion 2e of the second attachment leg 2c.

One end of the coil spring 6 is engaged with the slant portion 2i formed on the second attachment leg 2c, while the other end of the coil spring 6 is engaged with a spring stopper 9 mounted in the pin insertion hole 3j of the second pinching portion 3c.

At this time, turning of the second pinching portion 3c is limited by a pin 8 mounted in the pin insertion hole 2m provided in the attachment portion 2e of the second attachment leg 2c.

As shown in Figures 1 to 7, when the third pinching portion 4b of the lower pinching member 4 and the attachment portion 2e of the second attachment leg 2c are coupled to each other by the retaining member 7, the retaining member 7 is inserted in a coil spring 6 and the coil spring 6 is placed between the third pinching portion 4b and the attachment portion 2e of the second attachment leg 2c.

One end of the coil spring 6 is engaged with the slant portion 2i formed on the second attachment leg 2c, while the other end of the coil spring 6 is engaged with a spring stopper 9 mounted in the pin insertion hole 4j of the third pinching portion 4b.

The range of turning of the U-cut 4h of the third pinching portion 4b is limited by a pin 8 mounted in the pin insertion hole 2m bored in the attachment portion 2e of the second attachment leg 2c.

When the fourth pinching portion 4c of the lower pinching member 4 and the attachment portion 2d are coupled to each other by the retaining member 7, the retaining member 7 is inserted in a coil spring 6 and the coil spring 6 is placed between the fourth pinching portion 4c and the attachment portion 2d.

One end of the coil spring 6 is engaged with the slant portion 2h formed on the attachment portion 2d of the first attachment leg 2b, while the other end of the coil spring 6 is engaged with a spring stopper 9 mounted in the pin insertion hole 4j of the fourth pinching portion 4c.

The range of turning of the U-cut 4h of the fourth pinching portion 4c is limited by a pin 8 mounted in the pin insertion hole 2m bored in the attachment portion 2d of the first attachment leg 2b.

Accordingly, as shown in Figure 2, when the lower pinching member 4 is turned counterclockwise on the retaining member 7, the spring stopper 9 inserted in the pin insertion hole 4j and the slant portion 2h are brought closer to each other and the opposite ends of the coil spring 6 are also brought closer to each other. A clockwise torque is then produced on the lower pinching member 4 by the restoring force of the coil spring 6 acting to return to the original state.

Thus, when the upper pinching member 3 and the lower pinching member 4 are coupled to the base member 2, the coil springs 6 are disposed to produce pinching force between the upper pinching member 3 and the lower pinching member 4, thereby enabling mount of the universal fixing metal fitting 1 for an operating table on an accessory attachment rail 11b by firmly pinching the accessory attachment rail 11b.

In the present invention, when the first pinching portion 3b and the second pinching portion 3c of the upper pinching member 3 and the attachment portion 2d of the first attachment leg 2b and the attachment portion 2e of the second attachment leg 2c are coupled to each other, two coil springs 6 in total are used each in a one-to-one relationship with the coupling position.

Also, when the third pinching portion 4b and the fourth pinching portion 4c of the lower pinching member 4 and the attachment portion 2d of the first attachment leg 2b and the attachment portion 2e of the second attachment leg 2c are coupled to each other, two coil springs 6 in total are used each in a one-to-one relationship with the coupling position.

The coil spring 6 may be used for coupling of only one of the first pinching portion 3b and the second pinching portion 3c.

Also, the coil spring 6 disposed and used at the time of coupling of the third pinching portion 4b and the fourth pinching portion 4c of the lower pinching member 4 may be disposed and used at both or only one of the third pinching portion 4b and the fourth pinching portion 4c.

That is, when the first pinching portion 3b and the second pinching portion 3c of the upper pinching member 3 and the third pinching portion 4b and the fourth pinching portion 4c of the lower pinching member 4 are coupled to the first attachment leg 2b and the second attachment leg 2c, four coil springs 6 may be used or one coil spring 6 used on the upper pinching member 3 side and another coil spring 6 used on the lower pinching member 4 side, two coil springs 6 in total, may be disposed and used.

Figure 13 is a diagram showing the state of use of the universal fixing metal fitting for an operating table according to the present invention. At the time of mounting on an operating table 11, the universal fixing metal fitting 1 for an operating table according to the present invention is first attached to an accessory attachment rail 11b provided parallel to an operating table side surface 11a. At the time of mounting, the U-cuts 3h and 4h of the upper pinching member 3 and the lower pinching member 4 of the universal fixing metal fitting 1 for the operating table are opened outwardly and the accessory attachment rail 11b is pinched therebetween for attachment. At this time, even after the external force applied to the universal fixing metal fitting 1 for the operating table has been removed, the fixing metal fitting 1 is fixed on the accessory attachment rail 11b by the torque produced on the spring stoppers 9 so as not to fall from the rail 11b.

A member to be mounted is inserted in the space 2j in the base member 2. The inserted member may have the shape of a plate or a round rod. An attachment rod 10 in the form of a rectangular plate is illustrated in Figure 13 by way of example. Description will be made below of a case of using the attachment rod 10. Side surfaces of the attachment rods 10 are fitted to the L-cuts 3d and 4d formed in the connecting portions 3a and 4a or to the V-cuts 3k and 4k formed inwardly relative to the L-cuts 3d and 4d, and the fastening portion 5 is rotated for fastening by forcing the screw-engaging shaft 5b in the direction of the fixing member.

The attachment rod 10 is thereby caused to push the upper pinching member 3 and the lower pinching member 4 to produce a torque about the retaining members 7 such that the upper pinching member 3 and the lower pinching member 4 turn in such directions as to pinch the accessory attachment rail 11b.

In this way, the attachment rod 10 of the member to be mounted on the operating table 11 is firmly fixed on the accessory attachment rail 11b by the universal fixing metal fitting 1 for the operating table.

In the example of use shown in Figure 13, the appliance to be mounted on the operating table 11 is a trunk-fixing pad 10d. The trunk-fixing pad 10d is connected to a coupling part 10a by a connecting member 10b, and the coupling part 10a is attached to the upper end of the attachment rod 10 and fixed by an adjusting part 10c.

By fastening with the fastening portion 5, the appliance to be mounted is fixed on the base member 2 and the pinching force of the upper pinching member 3. Simultaneously, the lower pinching member 4 is increased by fastening with the fastening portion 5.

The universal fixing metal fitting 1 for the operating table is capable of being firmly fixed on the accessory attachment rail 11b of the operating table 11 and enabling the appliance to be mounted on the universal fixing metal fitting 1 for the operating table to be firmly fixed simultaneously.

### Industrial Applicability

The universal fixing metal fitting 1 for an operating table according to the present invention has advantages described below.

First, fixing between the universal fixing metal fitting for an operating table and an accessory attachment rail and fixing between the universal fixing metal fitting for an operating table and an appliance to be fixed can be simultaneously performed. Thus, the universal fixing metal fitting can be easily mounted in a shorter time and can be firmly fixed.

Second, although a case where appliances having coupling portions varying in shape and state are to be fixed is conceivable, the universal fixing metal fitting for an operating table according to the present invention is capable of being adapted to different shapes such as the shape of a member having a square or rectangular section and the shape of a round rod and, therefore, capable of being used in various circumstances including changing the appliance to be fixed, and is particularly advantageous on a medical scene where safety is required and various appliances are used.

Third, while there has been a need to use different fixing metal fittings in a case where there are operating table accessory attachment rails differing in shape as between Japan and Europe, the present invention is adaptable to rails of any shape and can therefore be used even when the rail shape is changed.

Fourth, while an appliance to be fixed is ordinarily placed under a sterilized clean sheet with which a subject on an operating table is covered, or placed so as not to contact the sheet, the fixing metal fitting can be mounted on a rail even in a state where the rail is covered with such a sheet to become thicker relative to the normal state, because the fixing metal fitting can be sterilized by steam at a high pressure and can be used regardless of the rail shape.

## Claims

1. A universal fixing metal fitting for an operating table **characterized by** comprising a base member in which a first attachment leg having an attachment portion and including a slant portion formed thereon and a second attachment leg having an attachment portion and including a slant portion formed thereon are formed continuously with a flat portion having a screw-engaging hole formed at its center, an upper pinching member in which a first pinching portion is formed continuously with one end of a connecting portion having a V-cut at its center and L-cuts formed continuously with the V-cut, and in which a second pinching portion is formed continuously with the other end of the connecting portion, a lower pinching member in which a third pinching portion is formed continuously with one end of a connecting portion having a V-cut at its center and L-cuts formed continuously with the V-cut, and in which a fourth pinching portion is formed continuously with the other end of the connecting portion, and a fastening portion in which a handle having spherical stoppers provided on its opposite ends is loosely fitted in a through hole in a cylindrical head portion provided on one end of a screw-engaging shaft, the universal fixing metal fitting also **characterized in that** when the upper pinching member and the lower pinching member are coupled to the base member, coil springs are attached by being interposed between the first pinching portion and the attachment portion, between the second pinching portion and the attachment portion, between the third pinching portion and the attachment portion and between the fourth pinching portion and the attachment portion.

2. The universal fixing metal fitting for an operating table according to claim 1, **characterized in that** a pressing member is attached to a tip of the screw-engaging shaft.
